# FASCICULE DE BREVET EUROPEEN

(11) **EP 2 125 787 B1**
(45) Date de publication et mention de la délivrance du brevet: **01.03.2017**
(21) Numéro de dépôt: 08761774.2
(22) Date de dépôt: 17.01.2008
(51) Int. Cl.: C07D 401/14, A61K 31/4439, A61P 25/00, A61P 17/00, A61P 13/00, A61P 11/00

(54) **DERIVES DE N-(HETEROARYL)-1-HETEROARYL-1H-INDOLE-2-CARBOXAMIDES, LEUR PREPARATION ET LEUR APPLICATION EN THERAPEUTIQUE**
DERIVATE VON N-(HETEROARYL)-1-HETEROARYL-1H-INDOL-2-CARBOXAMIDEN SOWIE IHRE HERSTELLUNG UND THERAPEUTISCHE VERWENDUNG
DERIVATIVES OF N-(HETEROARYL)-1-HETEROARYL-1H-INDOL-2-CARBOXAMIDS, PREPARATION THEREOF AND THERAPEUTIC USE THEREOF

(30) Priorité: 19.01.2007 FR 0700357
(43) Date de publication de la demande: 02.12.2009
(73) Titulaire: SANOFI, 75008 Paris (FR)
(72) Inventeur: DUBOIS, Laurent, F-75013 Paris (FR); EVANNO, Yannick, F-75013 Paris (FR); MALANDA, André, F-75013 Paris (FR); MALOIZEL, Christian, F-75013 Paris (FR)
(74) Mandataire: Nony
(86) Numéro de dépôt international: PCT/FR2008/000055
(87) Numéro de publication internationale: WO 2008/107544

(56) Documents cités:
- WO-A-03/068749
- WO-A-2004/072069
- WO-A-2006/072736
- WO-A-2007/010144
- FR-A1- 2 874 015
- US-A1- 2005 165 049

## Description

L'invention a pour objet des composés dérivés de *N*-(hétéroaryl)-1-hétéroaryl-1*H*-indole-2-carboxamides, qui présentent une activité antagoniste *in vitro* et *in vivo* pour les récepteurs de type TRPV1 (ou VR1).

On connaît des documents FR 2874015 et WO 2007/010144 des dérivés de N-(1 H-indolyl)-1 H-indole-2-carboxamides et de N-(hétéroaryl)-1-hétéroarylalkyl-1H-indole-2-carboxamides qui présentent une activité antagoniste *in vitro* et *in vivo* pour les récepteurs de type TRPV1 (ou VR1).
Les demandes de brevet US 2005/0165049, WO 2004/072069 et WO 03/068749 enseignent également des dérivés carboxamides utiles comme antagonistes des récepteurs vanilloïde.

Un premier objet de l'invention concerne les composés répondant à la formule générale (I) ci-après.
Un autre objet de l'invention concerne des procédés de préparation des composés de formule générale (I).
Un autre objet de l'invention concerne l'utilisation des composés de formule générale (I) notamment dans des médicaments ou dans des compositions pharmaceutiques.

Les composés de l'invention répondent à la formule générale (I) : 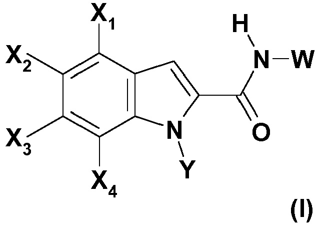 dans laquelle
X₁, X₂, X₃, X₄ représentent, indépendamment l'un de l'autre, un atome d'hydrogène ou d'halogène;
W représente un groupe bicyclique fusionné de formule : 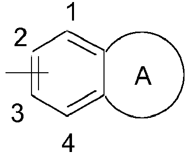 lié à l'atome d'azote par les positions 1, 2, 3 ou 4 ;
et W est choisi parmi les groupes benzimidazolyle et indolyle ;
le ou les atomes de carbone de A étant éventuellement substitués par un ou plusieurs groupes C₁-C₆-alkyle;
le ou les atomes d'azote de A étant éventuellement substitués par R₇, R₇ représentant un groupe C₁-C₆-alkyle ;
Y représente un pyridinyl éventuellement substitué par un ou plusieurs groupes C₁-C₆-alkyle à l'état de base ou de sel d'addition à un acide, ainsi qu'à l'état d'hydrate ou de solvate.

Dans les composés de formule générale (I), le ou les atomes d'azote de l'hétérocycle A ou de l'hétéroaryle Y peuvent être sous forme oxydée (N-oxyde).

Parmi les composés de formule générale (I) objets de l'invention, un premier sous-groupe de composés est constitué par les composés pour lesquels :
W est choisi parmi les groupes benzimidazol-5-yle et indol-5-yle ; et/ou
le ou les atomes de carbone de A étant éventuellement substitués par un ou plusieurs groupes C₁-C₆-alkyle, plus particulièrement un méthyle ; et/ou
le ou les atomes d'azote de A étant éventuellement substitués par R₇, R₇ représentant un groupe C₁-C₆-alkyle, plus particulièrement un méthyle.

La présente demande décrit aussi les composés de formule générale (I) pour lesquels : X₁, X₂, X₃, X₄ représentent, indépendamment l'un de l'autre, un atome d'hydrogène ou d'halogène ou un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₁-C₆-fluoroalcoxyle, cyano, C(O)NR₁R₂, nitro, NR₁R₂, C₁-C₆-thioalkyle, -S(O)-C₁-C₆-alkyle, -S(O)₂-C₁-C₆-alkyle, SO₂NR₁R₂, NR₃COR₄, NR₃SO₂R₅.

Parmi les composés de formule générale (I) objets de l'invention, un deuxième sous-groupe de composés est constitué par les composés pour lesquels :
X₁, X₃ et X₄ représentent un atome d'hydrogène.

Parmi les composés de formule générale (I) objets de l'invention, un troisième sous-groupe de composés est constitué par les composés pour lesquels :
X₁, X₃ et X₄ représentent un atome d'hydrogène et X₂ représente un atome d'halogène, plus particulièrement de fluor.

La demande décrit aussi les composés de formule générale (I) pour lesquels :
W représente un groupe bicyclique fusionné de formule : 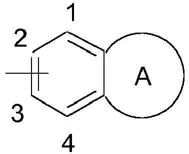 lié à l'atome d'azote par les positions 1, 2, 3 ou 4 ;
et W est choisi parmi les groupes indolyle, isoindolyle, indolinyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[1,4-*b*]diazépinyle, tétrahydrobenzo[1,4-e]diazépinyle, tétrahydrobenzo[1,4-*b*]oxazépinyle, tétrahydrobenzo[1,4-*b*]thiazépinyle ;
le ou les atomes de carbone et/ou d'azote dudit groupe W étant éventuellement substitués comme défini dans la formule générale (I).

Parmi les composés de formule générale (I) objets de l'invention, un quatrième sous-groupe de composés est constitué par les composés pour lesquels :
W représente un groupe indolyle ; le ou les atomes de carbone et/ou d'azote dudit groupe W étant éventuellement substitués comme défini dans la formule générale (I).

Parmi les composés de formule générale (I) objets de l'invention, un cinquième sous-groupe de composés est constitué par les composés pour lesquels :
Y représente un pyridinyle éventuellement substitué par un méthyle.

Les composés pour lesquels à la fois X₁, X₂, X₃, X₄, W et Y sont tels que définis dans les sous-groupes de composés ci-dessus, forment un sixième sous-groupe.

Dans le cadre de la présente invention on entend par :
- Cₜ-C_{z} où t et z peuvent prendre les valeurs de 1 à 7, une chaîne carbonée pouvant avoir de t à z atomes de carbone, par exemple C₁-C₃ une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;

- un alkylène : un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- un alkylénoxy : un alkylène comportant un atome d'oxygène ;
- un cycloalkyle : un groupe carboné cyclique. A titre d'exemples, on peut citer les groupes cyclopropyle, cyclobutyle, cyclopentyle, cyclohexyle, etc ;
- un fluoroalkyle : un groupe alkyle dont un ou plusieurs atomes d'hydrogène ont été substitués par un atome de fluor ;
- un alcoxyle : un radical -O-alkyle où le groupe alkyle est tel que précédemment défini ;
- un cycloalcoxyle : un radical -O-cycloalkyle où le groupe cycloalkyle est tel que précédemment défini ;
- un fluoroalcoxyle : un groupe alcoxyle dont un ou plusieurs atomes d'hydrogène ont été substitués par un atome de fluor ;
- un thioalkyle : un radical -S-alkyle où le groupe alkyle est tel que précédemment défini ;
- un thiofluoroalkyle : un groupe thioalkyle dont un ou plusieurs atomes d'hydrogène ont été substitués par un atome de fluor ;
- un aryle : un groupe aromatique cyclique comprenant entre 6 et 10 atomes de carbones. A titre d'exemples de groupes aryles, on peut citer les groupes phényle ou naphtyle ;
- un hétérocycle : un groupe aromatique, partiellement insaturé ou saturé de 5 à 12 chaînons, comprenant de un à cinq hétéroatomes choisis parmi O, S ou N.

A titre d'exemples d'hétérocycle, on peut citer les groupes azétidinyle, pyrrolidinyle, pipéridinyle, azépinyle, morpholinyle, thiomorpholinyle, pipérazinyle, homopipérazinyle, dihydro-oxazolyle, dihydrothiazolyle, dihydroimidazolyle, dihydropyrrolyle ou tétrahydropyridinyle, imidazolyle, pyrazolyle thiazolyle, oxazolyle, isothiazolyle, isoxazolyle, furanyle, thiophényle, oxadiazolyle, thiadiazolyle, triazole, tétrazolyle, pyridinyle, pyrazinyle, pyrimidinyle, pyridazinyle, triazinyle. - un hétéroaryle : un groupe hétérocyclique mono-, bi- ou tricyclique aromatique de 5 à 14 chaînons contenant de 1 à 8 hétéroatomes choisis parmi O, S ou N.

A titre d'exemples d'hétéroaryle monocyclique, on peut citer les groupes imidazolyle, pyrazolyle, thiazolyle, oxazolyle, isothiazolyle, isoxazolyle, furanyle, thiophényle, oxadiazolyle, thiadiazolyle, triazole, tétrazolyle, pyridinyle, pyrazinyle, pyrimidinyle, pyridazinyle, triazinyle.

A titre d'exemples d'hétéroaryle bicyclique, on peut citer les groupes indolyle, isoindolyle, benzofuranyle, benzothiophényle, benzoxazolyle, benzimidazolyle, indazolyle, benzothiazolyle, isobenzofuranyle, isobenzothiazolyle, isoquinoléinyle, pyrrolo[2,3-*c*]pyridinyle, pyrrolo[2,3-*b*]pyridinyle, pyrrolo[3,2-*b*]pyridinyle, pyrrolo[3,2-*c*]pyridinyle, quinoléinyle, isoquinoléinyle, cinnolinyle, quinazolinyle ou quinoxalinyle.
A titre d'exemples d'hétéroaryle tricyclique, on peut citer les groupes pyrido[1,2-a]benzimidazolyle, thiazolo[1,2-*a*]benzimidazolyle ou imidazo[1,2-*a*]benzimidazolyle;
- un atome d'halogène : un fluor, un chlore, un brome ou un iode ;
- « oxo » signifie « =O » ;
- « thio » signifie « =S ».

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvates, à savoir sous forme d'associations ou de combinaisons avec une ou plusieurs molécules d'eau ou avec un solvant. De tels hydrates et solvates font également partie de l'invention.

Dans ce qui suit, on entend par groupe partant, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxyle activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

Conformément à l'invention on peut préparer les composés de formule générale (I) selon le procédé illustré par le schéma 1 qui suit.

Selon le schéma 1, les composés de formule générale (IV) peuvent être obtenus par réaction d'un composé de formule générale (II) dans laquelle X₁, X₂, X₃, X₄ sont tels que définis dans la formule générale (I) et D représente un groupe C₁-C₆-alcoxyle, avec un composé de formule générale (III), dans laquelle Y est tel que défini dans la formule générale (I) et GP représente un groupe partant ou GP représente un groupe hydroxyle. La réaction peut être réalisée en présence d'une base telle que le carbonate de potassium ou du triphosphate de potassium, de préférence en présence d'une quantité catalytique d'un sel métallique tel que l'iodure de cuivre et d'un additif tel que la 1,2-diméthylaminocyclohexane (Buchwald S.L., J.Org.Chem. 2004, 69, 5578 - 5587).

Le composé de formule générale (I) est ensuite obtenu par réaction d'un composé de formule générale (IV), tel qu'obtenu ci-dessus, avec un amidure du composé de formule générale (V), dans laquelle W est tel que défini dans la formule générale (I), au reflux d'un solvant tel que le toluène. L'amidure du composé de formule générale (V) est préparé par action préalable du triméthylaluminium sur les amines de formule générale (V).

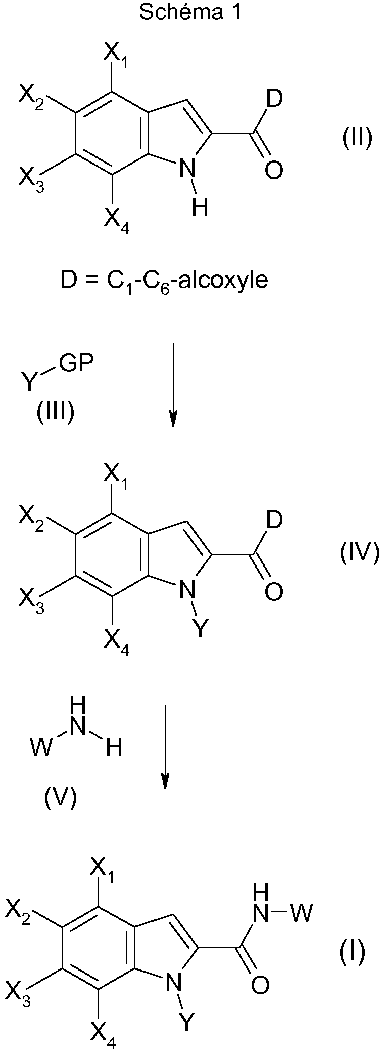

Les composés de formules générales (I), (II) et (IV), dans lesquelles X₁, X₂, X₃ et/ou X₄, représentent un groupe cyano ou un aryle, peuvent être obtenus par une réaction de couplage, catalysée par un métal tel que le palladium, réalisée sur les composés de formules générales (I), (II) et (IV) correspondants, dans lesquelles X₁, X₂, X₃ et /ou X₄, représentent un groupe partant , par exemple un brome, selon des méthodes qui sont décrites dans la littérature ou qui sont connues de l'homme de métier.

Les composés de formules générales (I), (II) et (IV), dans lesquelles X₁, X₂, X₃ et/ou X₄, représentent un groupe C(O)NR₁R₂, peuvent être obtenus à partir des composés de formules générales (I), (II) et (IV) correspondants, dans lesquelles X₁, X₂, X₃ et/ou X₄, représentent un groupe cyano, selon des méthodes qui sont décrites dans la littérature ou qui sont connues de l'homme du métier.

Les composés de formules générales (I), (II) et (IV), dans lesquelles X₁, X₂, X₃ et/ou X₄, représentent un groupe -S(O)-C₁-C₆-alkyle ou -S(O)₂-C₁-C₆-alkyle, peuvent être obtenus par oxydation des composés de formules générales (I), (II) et (IV) correspondants, dans lesquelles X₁, X₂, X₃ et/ou X₄, représentent un groupe C₁-C₆-thioalkyle, selon des méthodes qui sont décrites dans la littérature ou qui sont connues de l'homme du métier. Les composés de formules générales (I), (II) et (IV), dans lesquelles X₁, X₂, X₃ et/ou X₄, représentent un groupe NR₁R₂, NR₃COR₄ ou NR₃SO₂R₅, peuvent être obtenus à partir des composés de formules générales(I), (II) et (IV) correspondants, dans lesquelles X₁, X₂, X₃, et/ou X₄, représentent un groupe nitro, par exemple par réduction, puis acylation ou sulfonylation, selon des méthodes qui sont décrites dans la littérature ou qui sont connues de l'homme du métier.

Les composés de formules générales (I), (II) et (IV), dans lesquelles X₁, X₂, X₃ et/ou X₄, représentent un groupe NR₁R₂, NR₃COR₄ ou NR₃SO₂R₅, peuvent être obtenus à partir des composés de formules générales (I), (II) et (IV) correspondants, dans lesquelles X₁, X₂, X₃, et/ou X₄, représentent, par exemple, un atome de brome par réaction de couplage respectivement avec une amine, un amide ou une sulfonamide en présence d'une base, d'une phosphine et d'un catalyseur à base de palladium, selon des méthodes qui sont décrites dans la littérature ou qui sont connues de l'homme du métier.

Les composés de formules générales (I), (II) et (IV), dans lesquelles X₁, X₂, X₃ et/ou X₄, représentent un groupe SO₂NR₁R₂ peuvent être obtenus par une méthode analogue à celle décrite dans Pharmazie 1990, 45, 346, ou selon des méthodes qui sont décrites dans la littérature ou qui sont connues de l'homme du métier.

Les composés de formule générale (I), dans laquelle R₇ représente un atome d'hydrogène, peuvent être obtenus à partir des composés de formule générale (I) dans laquelle, par exemple, R₇ représente un groupe phénylméthyle, par hydrogénation catalysée, par le palladium par exemple, ou par toutes méthodes qui sont décrites dans la littérature ou qui sont connues de l'homme du métier.

Dans ce qui précède :
- les composés de formule générale (II) sont disponibles dans le commerce ou préparés selon de nombreux procédés décrits dans la littérature (D. Knittel Synthesis 1985, 2, 186 ; T.M. Williams J.Med.Chem. 1993, 36 (9), 1291 ; JP2001151771A2 par exemple) ;
- les composés de formule (III) sont disponibles dans le commerce ou accessibles en utilisant des méthodes connues de l'homme du métier ;
- les composés (V) et les autres réactifs, quand leur mode de préparation n'est pas décrit, sont disponibles dans le commerce ou décrits dans la littérature (WO03049702, WO03068749 par exemple).

L'exemple suivant décrit la préparation d'un composé conforme à l'invention. Cet exemple n'est pas limitatif et ne fait qu'illustrer la présente invention. Les numéros des composés exemplifiés renvoient à ceux donnés dans le tableau 1. Les microanalyses élémentaires, les analyses LC-MS (chromatographie liquide couplée à la spectrométrie de masse) les spectres I.R. et/ou R.M.N. confirment les structures des composés obtenus.

### Exemple 1 (Composé N°10)

### N-(1,2-diméthyl-1H-benzimidazol-5-yl)-5-fluoro-1-(4,6-diméthyl-pyridin-2-yl)-1H-indole-2-carboxamide

### 1.1 5-fluoro-1-(4,6-diméthyl-pyridin-2-yl)-1H-indole-2-carboxylate d'éthyle

On ajoute, dans un tube à pression, 4,35 g (21 mmoles) de 5-fluoro-1*H*-indole-2-carboxylate d'éthyle, 4,29 g (23,1 mmoles) de 2-bromo-4,6-diméthylpyridine, 9,36 g (44,1 mmoles) de triphosphate de potassium, 0,66 mL (4,2 mmoles) de 1,2-diméthylaminocyclohexane, 0.2 g (1,05 mmole) d'iodure de cuivre et 21 mL de toluène sec. Le tube est fermé et le mélange réactionnel est agité 5 jours à 110°C. Après ce temps le mélange est versé sur une solution de 100 mL d'eau et le pH du milieu est ajusté à pH 5 par ajouts d'acide acétique. On ajoute 100 mL d'acétate d'éthyle. La phase organique est séparée, lavée successivement avec 50 mL d'eau, 50 mL d'une solution aqueuse saturée en chlorure de sodium, puis séchée sur sulfate de sodium, filtrée puis concentrée sous pression réduite. On purifie le résidu par chromatographie préparative (éluant : dichlorométhane - acétate d'éthyle). On obtient 5.56 g de produit attendu qui est utilisé tel quel dans la suite de la synthèse.

### 1.2 N-(1,2-diméthyl-1H-benzimidazol-5-yl)-5-fluoro-1-(4,6-diméthyl-pyridin-2-yl)-1H-indole-2-carboxamide (composé n° 10)

On ajoute sous argon, à une solution de 0,34 g (2,11 mmoles) de 5-amino-1,2-diméthyl-1*H*-benzimidazole (WO2002059110) dans 19,2 mL de toluène sec, 1.54 mL de triméthylaluminium (2M dans le toluène). Après 15 minutes d'agitation à 50°C, on ajoute 0,6 g (1,92 mmole) de 5-fluoro-1-(4,6-diméthyl-pyridin-2-yl)-1*H*-indole-2-carboxylate d'éthyle, obtenu à l'étape 1.1. On porte le mélange réactionnel à reflux pendant 4 h puis on l'agite à température ambiante toute la nuit. On le verse sur 150 g de glace et 70 mL d'acétate d'éthyle. On sépare la phase aqueuse que l'on extrait par deux fois 30 mL d'acétate d'éthyle. Les phases organiques sont réunies, lavées successivement avec 50 mL de soude 1 N, deux fois avec 50 mL d'eau puis une fois avec 50 mL d'une solution saturée chlorure de sodium. Les phases organiques sont finalement séchées sur sulfate de sodium, filtrées puis concentrées sous pression réduite. On purifie le résidu par chromatographie préparative (éluant : dichlorométhane - méthanol). On obtient 0,71 g d'un solide qui est séché sous pression réduite.
PF : 130 - 140°C
R.M.N. ¹H (CDCl₃), δ (ppm): 2,4 (s, 3H) ; 2,57 (s, 3H) ; 2,58 (s, 3H) ; 3,71 (s, 3H) ; 7,15 (m, 7H) ; 7,6 (m, 2H) ; 8,7 (s, 1 H échangeable).

Les composés suivants ont été préparés par une méthode similaire à celle décrite dans l'exemple 1 :
- *N*-(1-méthyl-1*H*-indol-5-yl)-5-fluoro-1-(pyridin-4-yl)-1*H*-indole-2-carboxamide **(composé n°1)**
   R.M.N. ¹H (CDCl₃), δ (ppm): 3,70 (s, 3H) ; 6,33 (d, 1 H) ; 6,99 (m, 2H) ; 7,18 (m, 4H) ; 7,32 (m, 3H) ; 7,78 (s, 1 H) ; 7,89 (s, 1 H) ; 8,70 (s, 2H).
- *N*-(1-méthyl-1*H*-indol-5-yl)-5-fluoro-1-(pyridin-3-yl)-1*H*-indole-2-carboxamide **(composé n°2**)
   R.M.N. ¹H (CDCl₃), δ (ppm): 3,79 (s, 3H) ; 6,41 (d, 1 H) ; 7,09 (m, 3H) ; 7,21 (m, 3H) ; 7,42 (m, 2H) ; 7,82 (m, 3H) ; 8,7 (s, 2H).
- *N*-(1-méthyl-1*H*-indol-5-yl)-5-fluoro-1-(pyridin-2-yl)-1*H*-indole-2-carboxamide **(composé n°3**)
   R.M.N. ¹H (DMSO D₆), δ (ppm): 3,73 (s, 3H) ; 6,31 (d, 1H) ; 7,12 (txd, 1H) ; 7,29 (d, 1H) ; 7,48 (m, 7H) ; 7,89 (s, 1 H) ; 7,98 (txd, 1 H) ; 8,52 (m, 1 H) ; 10,41 (s, 1H).
- *N*-(1,2-diméthyl-1*H*-benzimidazol-5-yl)-5-fluoro-1-(pyridin-4-yl)-1*H*-indole-2-carboxamide **(composé n°4**)
   R.M.N. ¹H (DMSO D₆), δ (ppm): 2,50 (s, 3H) ; 3,72 (s, 3H) ; 7,19 (txd, 1H) ; 7,38 (m, 2H) ; 7,51 (m, 4H) ; 7,62 (d, 1 H) ; 7,88 (s, 1 H) ; 8,20 (d, 2H) ; 10,51 (s, 1 H).
- *N*-(1,2-diméthyl-1*H*-benzimidazol-5-yl)-5-fluoro-1-(pyridin-2-yl)-1*H*-indole-2-carboxamide **(composé n°5)**
   R.M.N. ¹H (DMSO D₆), δ (ppm): 2,50 (s, 3H) ; 3,70 (s, 3H) ; 7,11 (txd, 1 H) ; 7,45 (m, 7H) ; 7,84 (d, 1 H) ; 8,02 (txd, 1 H) ; 8,55 (d, 1 H) ; 10,49 (s, 1 H).
- *N*-(1,2-diméthyl-1*H*-benzimidazol-5-yl)-5-fluoro-1-(pyridin-3-yl)-1*H*-indole-2-carboxamide **(composé n°6**)
   R.M.N. ¹H (CDCl₃), δ (ppm): 2,55 (s, 3H) ; 3,72 (s, 3H) ; 7,05 (m, 2H) ; 7,21 (m, 2H) ; 7,41 (m, 2H) ; 7.60 (m, 2H) ; 7,80 (m, 1 H) ;8,02 (s, 1 H) ; 8,72 (m, 2H).
- *N*-(1-méthyl-1*H*-indol-5-yl)-5-fluoro-1-(4-méthylpyridin-2-yl)-1*H*-indole-2-carboxamide **(composé n°7)**
   R.M.N. ¹H (DMSO D₆), δ (ppm): 2,40 (s, 3H) ; 3,75 (s, 3H) ; 6,32 (d, 1 H) ; 7,11 (txd, 1 H) ; 7,34 (m, 7H) ; 7,53 (dxd, 1 H) ; 7,91 (s, 1 H) ; 8,39 (d, 1 H) ; 10,38 (s, 1 H).
- *N*-(1,2-diméthyl-1*H*-benzimidazol-5-yl)-5-fluoro-1-(4-méthylpyridin-2-yl)-1*H*-indole-2-carboxamide **(composé n°8**)
   R.M.N. ¹H (DMSO D₆), δ (ppm): 2,40 (s, 3H) ; 2,49 (s, 3H) ; 3,68 (s, 3H) ; 7,10 (txd, 1H) ; 7,24 (d, 1 H) ; 7,40 (m, 6H) ; 7,82 (s, 1 H) ; 8,38 (d, 1 H) ; 10,45 (s, 1H).
- *N*-(1-méthyl-1*H*-indol-5-yl)-5-fluoro-1-(2-méthylpyridin-4-yl)-1*H*-indole-2-carboxamide **(composé n°9**)
   R.M.N. ¹H (DMSO D₆), δ (ppm): 2,55 (s, 3H) ; 3,72 (s, 3H) ; 6,34 (d, 1H) ; 7,20 (m, 8H) ; 7,59 (dxd, 1 H) ; 7,89 (s, 1 H) ; 8,56 (d, 1 H) ; 10,34 (s, 1H).
- *N*-(1-méthyl-1*H*-indol-5-yl)-5-fluoro-1-(4,6-diméthyl-pyridin-2-yl)-1*H*-indole-2-carboxamide **(composé n°11**)
   R.M.N. ¹H (CDCl₃), δ (ppm): 2,41 (s, 3H) ; 2,58 (s, 3H) ; 3,78 (s, 3H) ; 6,41 (d, 1H) ; 7,05 (m, 4H) ; 7,3 (m, 5H) ; 7,86 (s, 1 H) ; 8,58 (s, 1 H).

Le tableau 1 qui suit illustre les structures chimiques et les propriétés physiques de quelques composés de formule générale (I) selon l'invention. Dans ce tableau la colonne "PF" renseigne les points de fusion des produits en degrés Celsius (°C).

**Tableau 1**

| | | | |
|---|---|---|---|
| 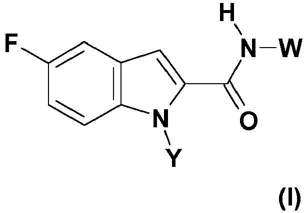 | | | |

| **N°** | **Y** | **W** | **PF (°C)** |
|---|---|---|---|
| 1 | pyridin-4-yle | 1-méthyl-indol-5-yle | 189 - 194 |
| 2 | pyridin-3-yle | 1-méthyl-indol-5-yle | 203 - 205 |
| 3 | pyridin-2-yle | 1-méthyl-indol-5-yle | 210 - 211 |
| 4 | pyridin-4-yle | 1,2-diméthyl-benzimidazol-5-yle | 226 - 229 |
| 5 | pyridin-2-yle | 1,2-diméthyl-benzimidazol-5-yle | 260 - 261 |
| 6 | pyridin-3-yle | 1,2-diméthyl-benzimidazol-5-yle | 139 - 141 |
| 7 | 4-méthyl-pyridin-2-yle | 1-méthyl-indol-5-yle | 276 - 279 |
| 8 | 4-méthyl-pyridin-2-yle | 1,2-diméthyl-benzimidazol-5-yle | 270 - 278 |
| 9 | 2-méthyl-pyridin-4-yle | 1-méthyl-indol-5-yle | 213 - 216 |
| 10 | 4,6-diméthyl-pyridin-2-yle | 1,2-diméthyl-benzimidazol-5-yle | 130 - 140 |
| 11 | 4,6-diméthyl-pyridin-2-yle | 1-méthyl-indol-5-yle | 198 - 199 |

Les composés de l'invention ont été soumis à des essais pharmacologiques *in vitro* qui ont mis en évidence leur intérêt comme substances à activités thérapeutiques.

### Test d'inhibition du courant induit par la capsaïcine sur les DRG de rat

- Culture primaire de cellules de ganglions de racine dorsale (DRG) de rat :
   Les neurones du DRG expriment naturellement le récepteur TRPV1.
      Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM glutamine, 100 µg/ml gentamicine et 50 ng/ml de NGF, puis déposées sur des lamelles de verre recouvertes de laminine (0.25 x 10⁶ cellules par lamelle) qui sont ensuite placées dans des boîtes 12 puits Corning. Les cellules sont incubées à 37°C en atmosphère humidifiée contenant 5% de CO₂ et 95% d'air. De la cytosine β-D-arabinoside (1 µM) est ajoutée 48 h après la mise en culture, pour prévenir le développement des cellules non neuronales. Les lamelles sont transférées dans les chambres expérimentales pour les études de patch-clamp après 7-10 jours de culture.
- Electrophysiologie :
   Les chambres de mesure (volume 800 µl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 ml/min) à l'aide d'un distributeur de solutions acceptant 8 entrées et dont la sortie unique, constituée par un tube de polyéthylène (ouverture 500µm) est placée à moins de 3 mm de la cellule étudiée. La configuration "cellule entière" de la technique de patch-clamp a été utilisée. Les pipettes en verre borosilicaté (résistance 5-10 MOhms) sont approchées de la cellule grâce à un micromanipulateur piézoélectrique 3D (Burleigh, PC1000) . Les courants globaux (potentiel de membrane fixé à -60 mV) sont enregistrés avec un amplificateur Axopatch 1 D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.

L'application d'une solution micromolaire de capsaïcine, provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés à tester sont appliqués seuls à une concentration donnée (concentration de 10 nM ou de 0,1 nM) pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.

Les pourcentages d'inhibition de la réponse capsaïcine (1 µM) sont compris entre 20% et 100% pour les composés les plus actifs de l'invention testés à la concentration de 10 nM à 0,1 nM (voir l'exemple du tableau 2).

Les composés de l'invention sont donc des antagonistes efficaces *in vitro* des récepteurs de type TRPV1.

**Tableau 2**

| **N°composé** | **% inhibition en patch DRG** |
|---|---|
| 10 | 70% (10 nM) |

Les résultats de ces essais montrent que les composés les plus actifs de l'invention bloquent la stimulation des récepteurs TRPV1.

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Ainsi, selon un autre de ses aspects, l'invention a pour objet des médicaments qui comprennent un composé de formule (I), ou un sel pharmaceutiquement acceptable, ou encore un hydrate ou un solvate dudit composé.

Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou iatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la nevralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénération, des accidents vasculaires hémorragiques (de la moelle et/ou du cerveau), de la douleur post-stroke.
Les composés de l'invention peuvent également être utilisés pour prévenir et/ou traiter les désordres métaboliques tels que le diabète.
Les composés de l'invention peuvent être utilisés pour la préparation d'un médicament destiné à prévenir et/ou à traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.
Les composés de l'invention peuvent être utilisés pour la préparation d'un médicament destiné à prévenir et/ou à traiter les désordres gynécologiques comme la vulvodynie, les douleurs liées aux salpingites, aux dysménorrhées.

On peut également utiliser ces produits pour la préparation d'un médicament destiné à prévenir et/ou à traiter les désordres gastrointestinaux tels que le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.
De même, les produits de la présente invention peuvent être utiles dans la prévention et/ou le traitement des désordres respiratoires tels que l'asthme, la toux, la pneumopathie obstructive chronique ou COPD (chronic obstructive pulmonary disease), la bronchoconstriction et les désordres inflammatoires. Ces produits peuvent également être utilisés pour prévenir et/ou traiter le psoriasis, le pruritis, les irritations dermiques, des yeux ou des muqueuses, l'herpès, le zona.
Les composés de l'invention peuvent également être utilisés pour traiter la dépression. Les composés de l'invention peuvent aussi être utilisés pour traiter les maladies du système nerveux central telles que la sclérose en plaques.

La présente invention vise également un composé de formule (I) pour son utilisation pour prévenir ou à traiter les pathologies dans lesquelles les récepteurs de type TRPV1 sont impliqués, choisies parmi la douleur, l'inflammation, les désordres métaboliques, les désordres urologiques, les désordres gynécologiques, les désordres gastro-intestinaux, les désordres respiratoires, le psoriasis, le pruritis, les irritations dermiques, des yeux ou des muqueuses, l'herpès, le zona, la sclérose en plaques et la dépression.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

II peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, décrit également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

## Revendications

1. Composé répondant à la formule générale (I) 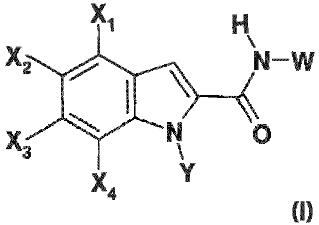 dans laquelle:
X₁, X₂, X₃, X₄ représentent, indépendamment l'un de l'autre, un atome d'hydrogène ou d'halogène;
W représente un groupe bicyclique fusionné de formule : 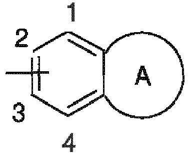 lié à l'atome d'azote par les positions 1, 2, 3 ou 4;
et W est choisi parmi les groupes benzimidazolyle et indolyle;
Le ou les atomes de carbone de A étant éventuellement substitués par un ou plusieurs groupes C₁-C₆-alkyle;
Le ou les atomes d'azote de A étant éventuellement substitués par R₇, R₇ représentant un groupe C₁-C₆-alkyle;
Y représente un pyridinyl éventuellement substitué par un ou plusieurs groupes C₁-C₆-alkyle;
à l'état de base ou de sel d'addition à un acide, ainsi qu'à l'état d'hydrate ou de solvate.

2. Composé de formule (I) selon la revendication 1, **caractérisé en ce que** W est choisi parmi les groups benzimidazol-5-yle et indol-5-yle; le ou les atomes de carbone de A étant éventuellement substitués par un ou plusieurs groupes C₁-C₆-alkyle; le ou les atomes d'azote de A étant éventuellement substitués par R₇, R₇ représentant un groupe C₁-C₆-alkye;
à l'état de base ou de sel d'addition à un acide, ainsi qu'à l'état d'hydrate ou de solvate.

3. Procédé de préparation d'un composé de formule (I) selon l'une quelconque des revendications 1 ou 2, **caractérisé en ce que** l'on fait réagir un composé de formule générale (IV) : 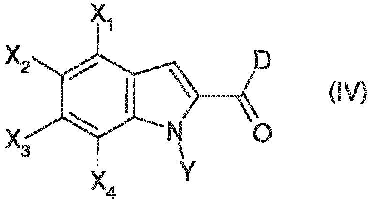 dans laquelle X₁, X₂, X₃, X₄ and Y sont tels que définis dans la formule générale (I) selon la revendication 1 et D représente un groupe C₁-C₆-alcoxyle,
avec un amidure du composé de formule générale (V) : 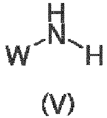 dans laquelle W est tel que défini dans la formule générale (I) selon la revendication 1, au reflux d'un solvant, l'amidure du composé de formule générale (V) étant préparé par action préalable du triméthylaluminium sur les amines de formule générale (V).

4. Médicament, **caractérisé en ce qu'**il comprend un composé de formule (I), selon l'une quelconque des revendications 1 ou 2, ou un sel pharmaceutiquement acceptable, ou encore un hydrate ou un solvate du composé de formule (I).

5. Composition pharmaceutique, **caractérisée en ce qu'**elle comprend un composé de formule (I) selon l'une quelconque des revendications 1 ou 2, ou un sel pharmaceutiquement acceptable, un hydrate ou un solvate de ce composé, ainsi qu'au moins un excipient pharmaceutiquement acceptable.

6. Composé de formule (I) selon l'une quelconque des revendications 1 ou 2, pour son utilisation pour prévenir ou traiter les pathologies dans lesquelles les récepteurs de type TRPV1 sont impliqués, choisies parmi la douleur, l'inflammation, les désordres métaboliques, les désordres urologiques, les désordres gynécologiques, les désordres gastro-intestinaux, les désordres respiratoires, le psoriasis, le pruritis, les irritations dermiques, des yeux ou des muqueuses, l'herpès, le zona, la sclérose en plaques et la dépression.

## Patentansprüche

1. Verbindung der allgemeinen Formel (I) 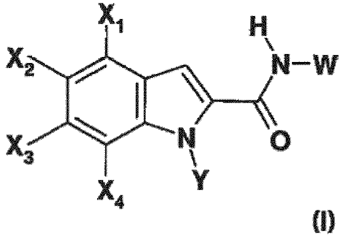 in der:
X₁, X₂, X₃ und X₄ unabhängig voneinander für ein Wasserstoff- oder Halogenatom stehen;
W für eine anellierte bicyclische Gruppe der Formel: 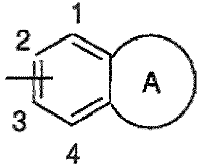 steht, die über die Positionen 1, 2, 3 oder 4 an das Stickstoffatom gebunden ist;
und W aus Benzimidazolyl- und Indolylgruppen ausgewählt ist;
wobei das Kohlenstoffatom bzw. die Kohlenstoffatome von A gegebenenfalls durch eine oder mehrere C₁-C₆-Alkylgruppen substituiert ist bzw. sind; wobei das Stickstoffatom bzw. die Stickstoffatome von A gegebenenfalls durch R₇ substituiert ist bzw. sind, wobei R₇ für eine C₁-C₆-Alkylgruppe steht;
Y für ein Pyridinyl, das gegebenenfalls durch eine oder mehrere C₁-C₆-Alkylgruppen substituiert ist, steht;
in Basen- oder Säureadditionssalzform sowie in Hydrat- oder Solvatform.

2. Verbindung der Formel (I) nach Anspruch 1, **dadurch gekennzeichnet, dass** W aus Benzimidazol-5-yl- und Indol-5-ylgruppen ausgewählt ist; wobei das Kohlenstoffatom bzw. die Kohlenstoffatome von A gegebenenfalls durch eine oder mehrere C₁-C₆-Alkylgruppen substituiert ist bzw. sind; wobei das Stickstoffatom bzw. die Stickstoffatome von A gegebenenfalls durch R₇ substituiert ist bzw. sind, wobei R₇ für eine C₁-C₆-Alkylgruppe steht;
in Basen- oder Säureadditionssalzform sowie in Hydrat- oder Solvatform.

3. Verfahren zur Herstellung einer Verbindung der Formel (I) nach einem der Ansprüche 1 oder 2, **dadurch gekennzeichnet, dass** man eine Verbindung der allgemeinen Formel (IV): 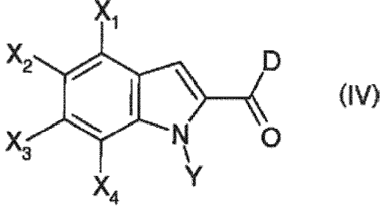 in der X₁, X₂, X₃, X₄ und Y wie in der allgemeinen Formel (I) gemäß Anspruch 1 definiert sind und D für eine C₁-C₆-Alkoxygruppe steht,
mit einem Amid der Verbindung der allgemeinen Formel (V): 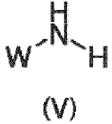 in der W wie in der allgemeinen Formel (I) gemäß Anspruch 1 definiert ist, am Rückfluss eines Lösungsmittels umsetzt, wobei das Amid der Verbindung der allgemeinen Formel (V) durch vorherige Einwirkung von Trimethylaluminium auf die Amine der allgemeinen Formel (V) hergestellt wird.

4. Arzneimittel, **dadurch gekennzeichnet, dass** es eine Verbindung der Formel (I) nach einem der Ansprüche 1 oder 2 oder ein pharmazeutisch annehmbares Salz oder auch ein Hydrat oder ein Solvat der Verbindung der Formel (I) umfasst.

5. Pharmazeutische Zusammensetzung, **dadurch gekennzeichnet, dass** sie eine Verbindung der Formel (I) nach einem der Ansprüche 1 oder 2 oder ein pharmazeutisch annehmbares Salz, ein Hydrat oder ein Solvat dieser Verbindung sowie mindestens einen pharmazeutisch annehmbaren Hilfsstoff umfasst.

6. Verbindung der Formel (I) nach einem der Ansprüche 1 oder 2 zur Verwendung zur Prävention oder Behandlung von Pathologien, an denen Rezeptoren vom TRPV1-Typ beteiligt sind, ausgewählt aus Schmerzen, Entzündungen, Stoffwechselstörungen, urologischen Störungen, gynäkologischen Störungen, gastrointestinalen Störungen, respiratorischen Störungen, Psoriasis, Pruritis, Haut-, Augen- oder Schleimhautreizungen, Herpes, Zona, multipler Sklerose und Depression.

## Claims

1. Compound corresponding to the general formula (I) 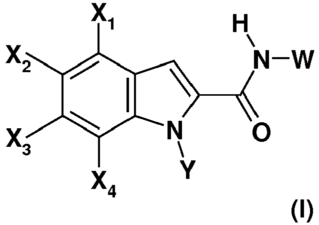 in which:
X₁, X₂, X₃, X₄ represent, independently of one another, a hydrogen or halogen atom;
W represents a fused bicyclic group of formula: 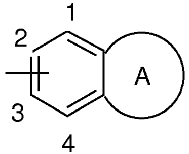 bonded to the nitrogen atom via the 1, 2, 3 or 4 positions;
and W is chosen from the benzimidazolyl and indolyl groups;
the carbon atom or atoms of A optionally being substituted by one or more C₁-C₆-alkyl groups;
the nitrogen atom or atoms of A optionally being substituted by R₇, R₇ representing a C₁- C₆- alkyl group;
Y represents a pyridinyl optionally substituted by one or more C₁-C₆-alkyl groups;
in the base form or in the form of an addition salt with an acid, and also in the hydrate or solvate form.

2. Compound of formula (I) according to Claim 1, **characterized in that** W is chosen from the benzimidazol-5-yl and indol-5-yl groups; the carbon atom or atoms of A optionally being substituted by one or more C₁-C₆-alkyl groups; the nitrogen atom or atoms of A optionally being substituted by R₇, R₇ representing a C₁-C₆-alkyl group;
in the base form or in the form of an addition salt with an acid, and also in the hydrate or solvate form.

3. Process for the preparation of a compound of formula (I) according to either one of Claims 1 and 2, **characterized in that** a compound of general formula (IV) : 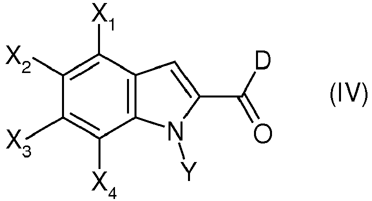 in which X₁, X₂, X₃, X₄ and Y are as defined in the general formula (I) according to Claim 1 and D represents a C₁-C₆-alkoxyl group,
is reacted with an amide of the compound of general formula (V): 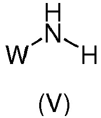 in which W is as defined in the general formula (I) according to Claim 1,
at reflux of a solvent, the amide of the compound of general formula (V) being prepared by prior action of trimethylaluminium on the amines on general formula (V).

4. Medicament, **characterized in that** it comprises a compound of formula (I) according to either one of Claims 1 and 2 or a pharmaceutically acceptable salt or also a hydrate or a solvate of the compound of formula (I).

5. Pharmaceutical composition, **characterized in that** it comprises a compound of formula (I) according to either one of Claims 1 and 2 or a pharmaceutically acceptable salt, a hydrate or a solvate of this compound, and also at least one pharmaceutically acceptable excipient.

6. Compound of formula (I) according to either one of Claims 1 and 2 for use thereof to prevent or to treat pathologies in which receptors of TRPV1 type are involved, chosen from pain, inflammation, metabolic disorders, urological disorders, gynaecological disorders, gastrointestinal disorders, respiratory disorders, psoriasis, pruritis, irritation of the skin, eyes or mucous membranes, herpes, shingles, multiple sclerosis and depression.
